# EUROPÄISCHE PATENTSCHRIFT

(11) **EP 1 900 717 B1**
(45) Veröffentlichungstag und Bekanntmachung des Hinweises auf die Patenterteilung: **13.07.2011**
(21) Anmeldenummer: 07115924.8
(22) Anmeldetag: 07.09.2007
(51) Int. Cl.: C07C 29/80, C07C 29/88, C07C 29/94, C07F 9/00

(54) **Verfahren zur Herstellung von hochreinen Niobalkoxiden**
Method for manufacturing extremely pure niobium alkoxides
Procédé destiné à la fabrication de niobalkoxides très purs

(30) Priorität: 14.09.2006 DE 102006043042
(43) Veröffentlichungstag der Anmeldung: 19.03.2008
(73) Patentinhaber: Heraeus Clevios GmbH, 63450 Hanau (DE)
(72) Erfinder: Reuter, Knud, 47800, Krefeld (DE)
(74) Vertreter: Herzog, Martin

(56) Entgegenhaltungen:
- EP-A- 1 764 354
- WO-A-02/074473
- BRADLEY D C ET AL: "Normal Alkoxides of Quinquevivalent Niobium" JOURNAL OF THE CHEMICAL SOCIETY, CHEMICAL SOCIETY. LETCHWORTH, GB, 1956, Seiten 2381-2384, XP002206709 ISSN: 0368-1769

## Beschreibung

Die Erfindung betrifft ein neues Verfahren zur Herstellung von hochreinen Niobalkoxiden, insbesondere von Niobethoxid.

Niobalkoxide, in der Literatur häufig auch als Niobalkoholate bezeichnet, lassen sich zur Abscheidung entsprechender Metalloxidschichten mittels Chemical Vapour Deposition (CVD) nutzen und sind daher wertvolle Ausgangsverbindungen zur Herstellung äußerst widerstandsfähiger Bauteile, die beispielsweise Verwendung in der Elektronikindustrie finden. Solche Metalloxidschichten lassen sich auch über die Hydrolyse nach der Sol-Gel-Methode aus den entsprechenden Niobalkoxiden herstellen. Die sehr hohe Dielektrizitätskonstante ermöglicht z.B. die Verwendung von Nioboxidschichten in sog. DRAMs (Dynamic Random Access Read/Write Memories). Auch in der Lampenindustrie spielt durch die CVD-Methodik abgeschiedenes Nioboxid eine Rolle, z. B. für die Herstellung von Glühbirnen.

Allerdings besteht ein Problem für die Elektronikindustrie und auch für Lampenhersteller in extremen Anforderungen an die Reinheit der Ausgangsmaterialien für solche Schichten, d.h. den Alkoxiden.

Die gebräuchlichste, technisch einfachste und wirtschaftlichste Herstellung der Niobalkoxide geht von den entsprechenden Metall-V-Chloriden und Alkoholen aus. Einen umfassenden Überblick gibt das Werk "Alkoxo and Aryloxo Derivatives of Metals" von D. C. Bradley, R. C. Mehrotra, I. P. Rothwell und A. Singh, Academic Press, 2001. Eine typische Vorgehensweise ist z.B. in der DE 10 113 169 A1 beschrieben.

Trotz einer ansonsten großen chemischen Ähnlichkeit sind Niobalkoxide, insbesondere Niobethoxid und höhere Niobalkoxide, im Gegensatz zu den praktisch farblosen analogen Tantalverbindungen nur äußerst schwer in einer hellfarbigen und damit hochreinen Form zu erhalten. In der Regel sind selbst destillierte Materialien gelb(orange) bis rotbraun gefärbt. Beispielsweise wird an typischem kommerziell vertriebenem Niobethoxid eine Hazen-Farbzahl von > 280 gemessen. Dies ist sogar nach wiederholtem Destillieren der Fall. Insbesondere ist eine zuverlässige, reproduzierbare Herstellung hellfarbigen oder praktisch farblosen Niobethoxids durch Destillation alleine (Vakuumdestillation; Sdp. 153 °C/0,1 mmHg bzw. 200 °C/5,5 mmHg) bisher nicht möglich.

Beispielsweise werden in D. C. Bradley, B. N. Chakravarti, W. Wardlaw; J. Chem. Soc. 1956, S. 2381 - 2384 Niobethoxid, Niob-n-propoxid und Niob-n-butoxid als gelbe Flüssigkeiten bezeichnet. Am Beispiel des Niobethoxids ist ausgeführt, dass die gelbe Farbe bei wiederholten Destillationen nicht zu beseitigen ist. Als Weg zu einem hochreinen, nur noch schwach gelben Niobethoxid wird folgendes aufwendige Verfahren beschrieben: Gelbes Niobethoxid wird in siedendem Isopropanol in ein kristallines Gemisch aus Nb(OEt)(OⁱPr)₄ und Nb(OEt)₂(OⁱPr)₃ umgewandelt, das viermal umkristallisiert wird. Danach wird dieses gemischte Ethoxid-Isopropoxid viermal mit Ethanol behandelt. Erst die Destillation des auf diese aufwendige Art erhaltenen Niobethoxids ergibt ein hochreines, nur noch schwach gelbes Produkt.

Ein derart kompliziertes Verfahren ist für eine technische Herstellung von hochreinem, fast farblosen Niobethoxid oder höheren Niobalkoxiden unbrauchbar.

Aufgabe der vorliegenden Erfindung ist es daher, Niobalkoxide, insbesondere Niobethoxid, Niob-n-propoxid, die isomeren Niobbutoxide sowie die isomeren Niobpentoxide, durch eine einfache, technisch leicht realisierbare und kostengünstige Methode mit sehr guter Reproduzierbarkeit in hochreiner, hellfarbiger Form bereitzustellen.

Diese Aufgabe wird durch das erfindungsgemäße Verfahren in überraschender Weise gelöst.

Gegenstand der Erfindung ist ein Verfahren zur Herstellung von hochreinen, hellfarbigen Niobalkoxiden mit einer Hazen-Farbzahl von <150 der allgemeinen Formel (I),

Nb(OR)₅ (I)

wobei
- R: für gleiches lineares oder verzweigtes C₁-C₁₂-Alkyl, bevorzugt lineares oder verzweigtes C₁-C₆-Alkyl, besonders bevorzugt für Methyl, Ethyl, n-Propyl, iso-Propyl, n-Butyl, iso-Butyl, 2-Butyl, tert-Butyl, n-Pentyl, 2-Pentyl, 3-Pentyl, 2-Methyl-1-butyl, 2-Methyl-2-butyl oder 3-Methyl-2-butyl steht,
dadurch gekennzeichnet, dass rohe Niobalkoxide der allgemeinen Formel (I) durch Durchleiten mit trockener Luft oder anderen trockenen, sauerstoffhaltigen Gasgemischen aus Sauerstoff und inerten Komponenten aus Edelgasen
und gegebenenfalls anschließende Destillation aufgereinigt werden.

Unter rohen Niobalkoxiden der allgemeinen Formel (I) werden im Rahmen der Erfindung solche Niobalkoxide der allgemeinen Formel (I) verstanden, die nach der Synthese dieser Niobalkoxide der allgemeinen Formel (I) gegebenenfalls nach Destillation oder gegebenenfalls auch ohne weitere Reinigung erhalten werden. Solche Syntheseverfahren sind dem Fachmann hinreichend bekannt und in der Literatur und Patentliteratur vielfach beschrieben, siehe z.B. in "Alkoxo and Aryloxo Derivatives of Metals" von D. C. Bradley, R. C. Mehrotra, I. P. Rothwell und A. Singh, Academic Press, 2001.

Färbende Verunreinigungen spielen erfahrungsgemäß bei Niobmethoxid eine geringere Rolle, da es bei sorgfältiger Arbeitsweise meist in Form farbloser (weißer) Kristalle anfällt. Daher stehen in bevorzugten Ausführungsformen des erfindungsgemäßen Verfahrens R und R¹ unabhängig voneinander für gleiche oder unterschiedliche, bevorzugt jedoch gleiche lineare oder verzweigte C₂ bis C₅-Alkylreste, ganz besonders bevorzugt für Ethyl, n-Propyl, iso-Propyl, n-Butyl, iso-Butyl, 2-Butyl, tert-Butyl, n-Pentyl, 2-Pentyl, 3-Pentyl, 2-Methyl-1-butyl, 2-Methyl-2-butyl oder 3-Methyl-2-butyl.

In einer ganz besonders bevorzugten Ausführungsform des erfindungsgemäßen Verfahrens sind das Niobalkoxid der allgemeinen Formel (I) Niobethoxid Ethanol.

Bei den sauerstoffhaltigen Gasgemischen handelt es sich vorzugsweise um solche, die neben Sauerstoff im Wesentlichen inerte Komponenten, wie z.B. Edelgase - beispielsweise Argon - und/oder Stickstoff enthalten, die zur Vermeidung der Hydrolyse des Niobalkoxids trocken sein sollten. Besonders bevorzugt erfolgt die Behandlung mit trockener Luft oder einem trockenem Sauerstoff-Argon-Gasgemisch.

Unter trocken sind im Rahmen der vorliegenden Erfindung Luft oder Gasgemische zu verstehen, die keine nennenswerte Hydrolyse der zu reinigenden Niobalkoxide bewirken. Bevorzugt wird unter trocken ein Wassergehalt entsprechend einem Wasserdampfdruck bei 20 °C von < 0,25 kPa, insbesondere < 0,05 kPa verstanden.

Wird unzureichend getrocknete Luft bzw. ein nicht ganz trockenes sauerstoffhaltiges Gasgemisch in das erfindungsgemäße Verfahren eingesetzt, so wird dennoch der gewünschte Reinigungseffekt erzielt. Dabei hydrolysiert die enthaltende Feuchtigkeit das Niobalkoxid der allgemeinen Formel (I) zu Alkohol ROH und Nioboxid. Der gebildete Alkohol sorgt zusätzlich zum enthaltenen Sauerstoff für den gewünschten Reinigungseffekt gemäß erfindungsgemäßem Verfahren. Diese Vorgehensweise ist jedoch nicht bevorzugt, da die gebildete Alkoholmenge nur sehr schlecht steuerbar und kontrollierbar ist und außerdem das gebildete Nioboxid ausfällt, damit verloren geht und zudem aufwendig, z. B. durch Filtration, wieder entfernt werden muss.

Bevorzugt ist demnach die Behandlung des rohen Niobalkoxids der allgemeinen Formel (I) mit trockener Luft oder einem trockenen sauerstoffhaltigen Gasgemisch. Besonders bevorzugt ist aus Kostengründen die Behandlung mit trockener Luft.

Die Bereitstellung trockener Luft oder eines trockenen Gasgemisches, beispielsweise Sauerstoff-Schutzgas(Argon)-Gemisches, erfolgt in der dem Fachmann bekannten Weise mittels Durchleiten durch flüssige oder bevorzugt Überleiten über feste Trockenmittel. Die Art der Trocknungsmittel ist insofern zu beachten, als sie nicht reduzierend wirken dürfen, um eine Reaktion mit dem Sauerstoffanteil im Gasgemisch zu vermeiden. Nicht reduzierende, geeignete Trockenmittel sind beispielsweise hygroskopische Salze wie CaCl₂, K₂CO₃, CuSO₄, MgClO₄, MgSO₄, Na₂SO₄, Metalloxide wie BaO, CaO, MgO, Säuren wie H₂SO₄, Säureanhydride wie P₂O₅, Molekularsiebe oder Kieselgel. Solche Trockenmittel sind dem Fachmann bekannt bzw. leicht auszuwählen und werden beispielsweise in Römpps Chemie-Lexikon, 8. Aufl., S. 4370 - 4371 oder im "Organikum", 21. Aufl., Wiley-VCH 2001, S. 24 beschrieben.

Die Variante des erfindungsgemäßen Verfahrens, bei der das zu reinigende rohe Niobalkoxid mit Luft oder einem sauerstoffhaltigen Gasgemisch behandelt wird, kann gegenüber der Behandlung mit Alkoholen wegen der sehr einfachen Durchführbarkeit bevorzugt sein. Die Behandlung mit Luft oder einem sauerstoffhaltigen Gasgemisch kann bevorzugt mittels Durchleiten durch oder Überleiten über, bevorzugt aber mittels Durchleiten durch das flüssige Niobalkoxid oder auch durch eine Lösung des Niobalkoxids in einem geeigneten Lösungsmittel erfolgen. Sofern Luft oder sauerstoffhaltiges Gasgemisch nur über das flüssige oder gelöste Niobalkoxid übergeleitet werden, was zwar möglich, aber wegen der schlechteren, langsameren Durchmischung weniger bevorzugt ist, muss für eine wirksame Umwälzung des Niobalkoxids, z. B. durch Rühren, gesorgt werden.

Geeignete Lösungsmittel für das Niobalkoxid sind beispielsweise aliphatische lineare, verzweigte oder cyclische Kohlenwasserstoffe wie n-Pentan, n-Hexan, n-Heptan, Isooctan, Cyclohexan oder aromatische Kohlenwasserstoffe wie Toluol oder Xylol, oder Gemische solcher Lösungsmittel.

Die erfindungsgemäßen Reinigungsoperationen mit einem Alkohol R¹OH, Luft oder anderen sauerstoffhaltigen Gasgemischen werden bevorzugt in einem Temperaturbereich von 10 °C bis 70 °C, bevorzugt bei 20 °C bis 50 °C, besonders bevorzugt bei Raumtemperatur (20 - 25 °C) durchgeführt.

Die erforderliche Menge der zur Reinigung eingesetzten trockenen Luft kann gut über den Fortschritt der Farbverbesserung des Niobalkoxids ermittelt werden. Je nach Verfärbungsgrad (Hazen-Farbzahl) des zu reinigenden Produkts ist für Luft beispielsweise eine Zeit von 15 min bis 2 h, oft von 30 min bis 1 h, für die Durchleitung durch das Niobalkoxid ausreichend. Dabei werden beispielsweise 0,1 bis 10 ml/sec, bevorzugt 0,5 bis 5 ml/sec Luft durchgeleitet, entsprechend einer Gesamtmenge von 90 ml bis 72 1, bevorzugt 900 ml bis 18 1 Luft. Diese Angaben sind nur als Richtschnur zu verstehen, sie können - je nach Reinigungsbedarf und vorliegender Verfärbung - auch über- oder unterschritten werden.

Der Sauerstoffgehalt in Schutzgasen wie Argon, die im Gemisch mit zugesetztem Sauerstoff eingesetzt werden können, orientiert sich an dem von Luft. Gut einsetzbare Mischungen enthalten dann z. B. 2 bis 35 Vol-%, bevorzugt 5 bis 25 Vol-% Sauerstoff. Ein entsprechender geeigneter Sauerstoffgehalt kann auch durch Zumischen von Luft erhalten werden, sodass als Schutzgas ein Gemisch Argon/Stickstoff vorliegt bzw. ein Argon-Luft-Gemisch zur Reinigung eingesetzt wird.

An die Reinigungsoperationen mit Luft oder anderen sauerstoffhaltigen Gasgemischen gemäß erfindungsgemäßem Verfahren kann sich gegebenenfalls eine Destillation unter vermindertem Druck anschließen. Die Durchführung einer Destillation von Niobalkoxiden unter vermindertem Druck ist dem Fachmann bekannt, siehe auch D. C. Bradley, B. N. Chakravarti, W. Wardlaw; J. Chem. Soc. 1956, S. 2381 - 2384.

**C₂-C₅-Alkyl** steht im Rahmen der Erfindung beispielsweise für Ethyl, n-Propyl, iso-Propyl, n-Butyl, sec.-Butyl, tert.-Butyl, n-Pentyl, 2-Pentyl, 3-Pentyl, 2-Methylbutyl, 3-Methylbutyl, neoPentyl, 1,1-Dimethylpropyl, 1,2-Dimethylpropyl, **C₁-C₆-Alkyl** steht im Rahmen der Erfindung darüber hinaus beispielsweise für Methyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Etlxyl-2-methylpropyl oder 1-Methyl-2-ethylpropyl, **C₁-C₁₂-Alkyl** steht im Rahmen der Erfindung darüber hinaus beispielsweise für n-Heptyl, n-Octyl, n-Nonyl, n-Decyl und n-Dodecyl.

Mit dem erfindungsgemäßen Verfahren zur Aufreinigung von Niobalkoxiden sind unabhängig von der Vorbehandlung und der technischen Vorgeschichte oder allgemein vom Herstellungsverfahren des Niobalkoxids gute Erfolge zu erzielen.

Es kann jedoch von Vorteil sein, besonders halogenarme, vorzugsweise chloridarme rohe Niobalkoxide der allgemeinen Formel (I) in das erfindungsgemäße Reinigungsverfahren einzusetzen. Ein geeignetes Verfahren zur Herstellung solcher halogenidarmer, vorzugsweise chloridarmer roher Niobalkoxide wird beispielsweise in der noch nicht veröffentlichten deutschen Patentanmeldung DE 10 2005 052 444.3 beschrieben.

Dabei handelt es sich insbesondere um ein Verfahren zur Reinigung von Niobalkoxiden der allgemeinen Formel (I), wobei
- rohe Niobalkoxide mit einem Halogengehalt, insbesondere C1-Gehalt > 100 ppm, gegebenenfalls > 200 ppm, die als Verunreinigung mindestens 0,05 Gew.-%, bevorzugt 0,1 bis 10,0 Gew.-% ein- oder mehrkerniger halogenhaltiger Metallalkoxide enthalten,
- mit maximal 30 Gew.-%, bevorzugt 4 bis 12 Gew.-%, bezogen auf die Gesamtmenge des rohen Alkoxids, eines Alkohols R²OH, wobei R² unabhängig von R und R¹ eine der vorangehend für R und R¹ genannte Bedeutung hat, gemischt werden und
- anschließend oder gleichzeitig (z.B. nach vorherigem Lösen im Alkohol R²OH) ein Überschuss, bezogen auf die Menge an ein- oder mehrkernigen halogenhaltigen Metallalkoxiden, bevorzugt von 0,1 bis 5,0 Gew.-%, bezogen auf die Gesamtmenge des rohen Alkoxids, Ammoniak zudosiert wird.

Ein solches Reinigungsverfahren speziell zur Beseitigung von Halogenid-, vorzugsweise Chloridverunreinigungen kann dem erfindungsgemäßen Verfahren bevorzugt vorgeschaltet sein. Besonders bevorzugt wird dabei ein rohes Nioboxid für den Einsatz im erfindungsgemäßen Verfahren mit einem Cl-Gehalt von weniger als 100 Gew.-ppm erhalten.

In bevorzugten Ausführungsformen des erfindungsgemäßen Verfahrens wird demnach als zu reinigendes rohes Niobalkoxid ein solches mit einen Cl-Gehalt von weniger als 100 Gew.- ppm (als Chlorid bestimmt) eingesetzt. Der Chloridgehalt kann beispielsweise coulometrisch nach DIN 38 405 D-1 bestimmt werden. Orientierend sind auch colorimetrische Methoden geeignet wie diejenige, basierend auf der Umsetzung mit Quecksilber(II)-thiocyanat und anschließender Reaktion der freigesetzten Thiocyanat-Ionen mit Eisen(III)-ionen zu rotem Eisen(III)-thiocyanat. Ganz besonders bevorzugt im Rahmen der vorliegenden Erfindung zur Reinigung halogenidhaltiger, insbesondere chloridhaltiger Niobalkoxide ist eine Kombination des Verfahrens zur Verringerung des Halogenid-, vorzugsweise Chloridgehaltes und anschließende Behandlung mit einem Alkohol R¹OH oder Luft bzw. einem sauerstoffhaltigen Gasgemisch.

Die gemäß dem erfindungsgemäßen Verfahren aufgereinigten Niobalkoxide sind für das bloße Auge praktisch farblos oder weisen nur noch eine geringfügige, hellgelbe Färbung auf. Diese Färbung kann mittels Messung der Hazen-Farbzahl bestimmt werden. Vorzugsweise werden nach dem erfindungsgemäßen Verfahren Niobalkoxide mit einer Hazen-Farbzahl von < 150, besonders bevorzugt < 120, ganz besonders bevorzugt < 60 erhalten. Die Hazen-Farbzahl (Pt-Co-Farbzahl) wird nach DIN EN ISO 6271-1+2 bestimmt.

Solche nach dem erfindungsgemäßen Verfahren hergestellten hochreinen Niobalkoxide eignen sich beispielsweise hervorragend zur Abscheidung entsprechender Metalloxidschichten mittels Chemical Vapour Deposition (CVD) und sind daher wertvolle Ausgangsverbindungen zur Herstellung äußerst widerstandsfähiger Bauteile, die beispielsweise Verwendung in der Elektronikindustrie finden.

Die folgenden Beispiele dienen der beispielhaften Erläuterung der Erfindung und sind nicht als Beschränkung aufzufassen.

### Beispiele:

Die Hazen-Farbzahl (Pt-Co-Farbzahl) wurde stets nach DIN EN ISO 6271-1+2 bestimmt.

### Beispiel 1: Reinigung von Niobethoxid mit niedrigem Cl-Gehalt

### a) Herstellung von Niobethoxid mit niedrigem Cl-Gehalt

810 g (3 mol) Niobchlorid wurden in 600 ml trockenem Heptan suspendiert. Hierzu wurden innerhalb von 2 h 11 kg absolutiertes (abs.) Ethanol zudosiert, wobei durch Kühlung mit Wasser/Eis die Innentemperatur unter 40°C gehalten wurde. Es wurde 1 h bei 25 °C nachgerührt.

Danach wurden bei maximal 35 °C (Kühlung mit Wasser/Eis) 321 g (19,5 mol) Ammoniak innerhalb von 3 h eingeleitet. Danach wurde vom Ammoniumchlorid abfiltriert und Ethanol bei 20 mbar, zum Schluss im Hochvakuum bei < 1 mbar abdestilliert.

Zum erhaltenen rohen Ethoxid wurden 70 g abs. Ethanol gegeben; anschließend wurden bei 23 °C in 1 h 33,8 g (1,99 mol) Ammoniak eingeleitet. Nach 2 h Nachrühren bei 23 °C wurden 800 ml Hexan zugesetzt, das Gemisch filtriert und das abfiltrierte Ammoniumchlorids mit Hexan nachgewaschen. Die vereinigten Hexan-Lösungen wurden unter vermindertem Druck destilliert. Das nach Abdestillation des Hexans erhaltene Niobethoxid enthielt ca. 35 ppm Cl.

### b) Reinigung mittels Zugabe von Ethanol(nicht erfindungsgemäß)

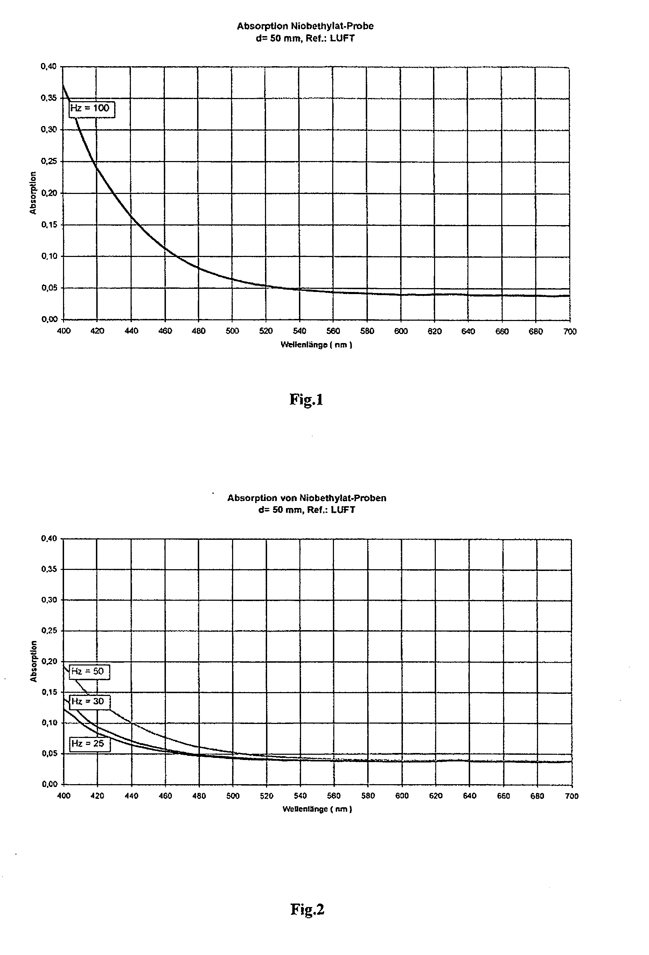

500 g rohes, undestilliertes Niobethoxid aus Beispiel 1 a) mit einem Cl-Gehalt von ca. 35 ppm mit orangegelber Farbe (Hazen-Farbzahl > 280) wurde mit 50 g trockenem Ethanol versetzt und 2 h unter Rühren auf 50 °C erhitzt. Anschließend wurde bei 150 °C/0,5 mbar destilliert. Das Destillat hatte eine gelbliche Farbe und eine Hazen-Farbzahl von 100; in Fig.1 ist die WellenlängenAbhängigkeit der Absorption dieser Niobethoxid-Probe dargestellt.

### Beispiel 2: Erfindungsgemäße Reinigung von Niobethoxid mittels Behandlung mit Luft

1,7 kg rohes Niobethoxid wurden bei 155 °C/0,6 mbar destilliert und ergaben ein orangegelbes Destillat (Hazen-Farbzahl > 280). Durch das Destillat wurde bei 23 °C 1 h lang über Caleiumehlorid getrocknete Luft geleitet. Danach hatte das Produkt eine gelbe Farbe und eine Hazen-Farbzahl von 145.

### Beispiel 3: Erfindungsgemäße Reinigung von Niobethoxid mit niedrigem C1-Gehalt mittels Behandlung mit Luft

Ein analog Beispiel 1a) hergestelltes rohes Niobethoxid wurde bei 150 °C/0,5 mbar destilliert. Das orangegelbe Niobethoxid-Destillat mit einem Cl-Gehalt von 20 ppm wurde wie in Beispiel 2 bei 23 °C 1 h mit über CaCl₂ getrockneter Luft behandelt. Die Farbe veränderte sich nach hellgelb.

### Beispiel 4: Erfindungsgemäße Reinigung von Niobethoxid mit niedrigem Cl-Gehalt mittels Behandlung mit Luft

### a) Herstellung von Niobethoxid mit niedrigem Cl-Gehalt

11,557 kg eines rohen, entsprechend 1 a) hergestellten Niobethoxids wurden mit 1156 g Ethanol und 57,8 g NH₃ ebenfalls wie in 1 a) zur Absenkung des Cl-Wertesbehandelt. Das erhaltene rohe Niobethoxid wies anschließend einen Cl-Gehalt von 35 ppm auf. Das Produkt wurde in 3 Portionen bei 150 °C / 0,5 mbar destilliert.

### b) Reinigung mittels Behandlung mit Luft

Durch jeweils 2 kg der drei verschiedenen Destillate aus 4 a) wurden jeweils ½ h lang mit einer Geschwindigkeit von ca. 50 ml/min über CaCl₂ getrocknete Luft geleitet. Die anschließende Messung der Hazen-Farbzahl der drei Niobethoxid-Portionen ergab Werte von 25, 30 und 50. Die Wellenlängenabhängigkeit zeigt Fig. 2.

## Patentansprüche

1. Verfahren zur Herstellung von hochreinen Niobalkoxiden der allgemeinen Formel (I),
Nb(OR)₅ x (1)
wobei
R für gleiches oder unterschiedliches, beverzeig aber gleiches lineares oder verzweigtes C₁-C₁₂-Alkyl steht,
**dadurch gekennzeichnet, dass** rohe Niobalkoxide der allgemeinen Formel (I)'durch
mit links Heisen-Franbzohl von < 150

2. Verfahren gemäß Anspruch 1, **dadurch gekennzeichnet, dass** R für lineares oder verzweigtes C₁-C₆-Alkyl

3. Verfahren gemäß Anspruch 1 oder 2, **dadurch gekennzeichnet, dass** R für lineares oder verzweigtes C₂-C₅-Alkyl

4. Verfahren gemäß wenigstens einem der Ansprüche 1 bis 3, **dadurch gekennzeichnet, dass** die rohen Niobalkoxide einen Cl-Gehalt von weniger als 100 Gew.-ppm hat.

5. Verfahren gemäß wenigstens einem der Ansprüche 1 bis 4, **dadurch gekennzeichnet, dass** die rohen Niobalkoxide der allgemeinen Formel (I) mit einem Halogengehal die als Verunreinigung mindestens 0,05 Gew.-% ein- oder mehrkentiger halogenhaltiger Metallalkoxide enthalten, vor dem Behandeln mit Luft oder anderen sauerstoffhaltigen Gasgemischen zur Entfernung von Halogen
• mit maximal 30 Gew.-% bezogen auf die Gesamtmenge des rohen Alkoxids, eines Alkohols R²OH, wobei R² unabhängig von R und R¹ eine der vorangehend für R genannte Bedeutung hat gemischt werden und
• anschließend oder gleichzeitig ein Überschuss, bezogen auf die Menge an ein- oder mehrkernigen halogenbaltigen Metallalkoxiden, Ammoniak zudosiert wird.

## Claims

1. Method for manufacturing extremely pure niobium alkoxides with a Hazen colour number of < 150, of the general formula (I)
Nb(OR)₅ (I)
wherein
R represents identical or different, but preferably identical linear or branched C₁-C₁₂-alkyl,
**characterized in that** crude niobium alkoxides of the general formula (I) are purified by treatment with dry air or other dry oxygen-containing gas mixtures of oxygen and inert components of noble gases.

2. Method according to claim 1, **characterized in that** R represents linear or branched C₁-C₆-alkyl.

3. Method according to claim 1 or 2, **characterized in that** R represents linear or branched C₂-C₅-alkyl.

4. Method according to at least one of claims 1 to 3, **characterized in that** the crude niobium alkoxides have a Cl content of less than 100 ppm by wt.

5. Method according to at least one of claims 1 to 4, **characterized in that** before the treatment with air or other oxygen-containing gas mixtures, the crude niobium alkoxides of the general formula (I) with a halogen content, which contain at least 0.05 wt.% of mono- or polynuclear halogen-containing metal alkoxides as an impurity,
- are mixed with a maximum of 30 wt.%, based on the total amount of the crude alkoxide, of an alcohol R²-OH, wherein R² independently of R and R¹ has one of the meanings given above for R, and
- subsequently or simultaneously an excess, based on the amount of mono- or polynuclear halogen-containing metal alkoxides, of ammonia is metered in,
in order to remove halogen.

## Revendications

1. Procédé de préparation d'alcoxydes de niobium ultrapurs ayant un numéro de couleur Hazen de < 150, de la formule générale
Nb(OR)₅ (I)
dans laquelle
R représente des alkyles en C₁ à C₁₂, identiques ou différents mais de préférence identiques, linéaires ou ramifiés,
**caractérisé en ce que** des alkoxydes de niobium bruts de la formule générale (I) sont purifiés par traitement avec de l'air sec ou un autre mélange de gaz sec contenant de l'oxygène, en oxygène et en des composants inertes en gaz rares

2. Procédé selon la revendication 1, **caractérisé en ce que** R représente un alkyle en C₁ à C₆ linéaire ou ramifié.

3. Procédé selon la revendication 1 ou 2, **caractérisé en ce que** R représente un alkyle en C₂ à C₅ linéaire ou ramifié.

4. Procédé selon l'une au moins des revendications 1 à 3, **caractérisé en ce que** les alkoxydes de niobium bruts ont une teneur en C1 de moins de 100 ppm en poids.

5. Procédé selon l'une au moins des revendications 1 à 4, **caractérisé en ce que** les alkoxides de niobium bruts de la formule générale (I) avec une teneur en halogène, qui contiennent comme impuretés au moins 0,05 % en poids en alcoxydes halogénés de métal à un ou plusieurs noyaux,
• sont mélangés, avant le traitement avec l'air ou d'autres mélanges de gaz oxygénés pour l'élimination de l'halogène, à 30 % en poids au maximum, rapportés à la quantité totale de l'alcoxyde brut, d'un alcool R²OH, R² ayant indépendamment de R et de R¹ une des significations citées ci-avant pour R et
• **en ce qu'**ensuite ou en même temps, un excès d'ammoniac, rapporté à la quantité d'alcoxydes halogénés à un ou plusieurs noyaux, est ajouté de façon dosée.
